# EUROPEAN PATENT SPECIFICATION

(11) **EP 3 851 022 B1**
(45) Date of publication and mention of the grant of the patent: **10.06.2026**
(21) Application number: 19859938.3
(22) Date of filing: 04.09.2019
(51) Int. Cl.: A61B 1/045, A61B 1/00

(54) **MEDICAL IMAGE PROCESSING APPARATUS, MEDICAL IMAGE PROCESSING PROGRAM, AND ENDOSCOPIC SYSTEM**
MEDIZINISCHE BILDVERARBEITUNGSVORRICHTUNG, MEDIZINISCHES BILDVERARBEITUNGSPROGRAMM UND ENDOSKOPSYSTEM
APPAREIL DE TRAITEMENT D'IMAGE MÉDICALE, PROGRAMME DE TRAITEMENT D'IMAGE MÉDICALE ET SYSTÈME ENDOSCOPIQUE

(30) Priority: 11.09.2018 JP 2018169398
(43) Date of publication of application: 21.07.2021
(73) Proprietor: FUJIFILM Corporation, Tokyo 106-8620 (JP)
(72) Inventor: KAMON, Shumpei, Ashigarakami-gun, Kanagawa 258-8538 (JP)
(74) Representative: Dehns Germany Partnerschaft mbB
(86) International application number: PCT/JP2019/034789
(87) International publication number: WO 2020/054541

(56) References cited:
- EP-A1- 3 795 063
- WO-A1-2007/135913
- WO-A1-2016/199273
- WO-A1-2017/073338
- JP-A- 2000 079 087
- JP-A- 2016 158 681

## Description

### BACKGROUND OF THE INVENTION

### 1. Field of the Invention

The present invention relates to a medical image processing apparatus, a program, and an endoscope system, and particularly to a technique for reporting a region of interest in a time-series image.

### 2. Description of the Related Art

In the medical field, inspections are performed by using an endoscope system. In recent years, it has been known that a time-series image (moving image) captured by an endoscope-scope is displayed on a monitor while a region of interest such as a lesion region included in the time-series image is recognized through image analysis of the time-series image and is reported to support inspections.

If a lesion is detected in the time-series image by a recognizer during an inspection using an endoscope, by surrounding the lesion region in the image displayed on the monitor by a frame, the lesion can be reported.

An endoscope image processing apparatus described in WO2017/073338A includes a reporting unit and an enhancement processing unit. If a feature region such as a lesion part is detected by a detection unit, the reporting unit generates a first display image for reporting that a feature region is detected. The enhancement processing unit generates a second display image obtained by performing enhancement processing on the feature region.

The first display image generated by the reporting unit is formed of an observation image and a reporting image (e.g., a flag) to be displayed in a display region different from a display region of the observation image. When a flag is set (reporting image is displayed) on the first display image, a surgeon can observe the observation image more carefully and can visually find a lesion part by themself.

According to the invention described in WO2017/073338A, by displaying the first display image formed of an observation image and a reporting image to be displayed in a display region different from a display region of the observation image, although observation of the observation image is not interrupted by the reporting image, it is not possible to immediately check the region where the lesion region is present in the observation image by using only the reporting image. On the other hand, if the first display image is switched to the second display image, the switching operation is complicated, and if the first display image and the second display image are to be displayed concurrently, the display regions of the first display image and the second display image are reduced.

In addition, JP2016-158681A displays a marking image formed by arranging a plurality of arrows so as to overlay the marking image and surround a region where a score indicating a degree of seriousness of a lesion in a tissue is high. However, the score is calculated for each pixel, and based on the calculation results, the plurality of arrows are arranged on a boundary of the high-score region. This increases the number of arrows, and it may be difficult in some cases to compare the region with a high degree of seriousness and its periphery with each other. EP3795063A1 is concerned with a medical image processing device, method and endoscope system. WO2016/199273A1 is concerned with an endoscope device and operation method for endoscope device. WO2017/073338A1 is concerned with an endoscope image processing device.

### SUMMARY OF THE INVENTION

If a lesion region in an observation image is surrounded by a frame image by using, for example, a square frame image as a marker image, as in the second display image described in WO2017/073338A, it may be difficult to be compared with its periphery.

The present invention has been made in view of such circumstances, and an object is to provide a medical image processing apparatus, a program, and an endoscope system that report a region of interest without interrupting observation of a time-series image.

In order to achieve the above object, a medical image processing apparatus according to an aspect is a medical image processing apparatus comprising: a coordinates calculating unit that, based on region-of-interest information indicating a region of interest in a time-series image, calculates one or more sets of coordinates of interest indicating a position of the region of interest in the time-series image; and a reporting information display control unit that, based on the one or more sets of coordinates of interest, superposes a figure on the time-series image, in which the reporting information display control unit increases a size of the figure and decreases a ratio of the size of the figure to a size of the region of interest as the size of the region of interest is larger.

According to this aspect, as the size of the region of interest is larger, the size of the figure is increased, and the ratio of the size of the figure to the size of the region of interest is decreased. Thus, even if the region of interest is large, the size of the figure does not become excessively large, and comparison between the large region of interest and its peripheral region is not interrupted. In addition, as the size of the region of interest is smaller, the size of the figure is decreased, and the ratio of the size of the figure to the size of the region of interest is increased. Thus, comparison between the small region of interest and its peripheral region is not interrupted. Accordingly, the region of interest can be reported without interrupting observation of the time-series image.

The medical image processing apparatus preferably further includes: a time-series image acquiring unit that acquires the time-series image including a photographic subject image; a region-of-interest detecting unit that detects the region of interest from the time-series image acquired by the time-series image acquiring unit; and a region-of-interest information acquiring unit that acquires region-of-interest information indicating the region of interest from the region-of-interest detecting unit. Thus, the time-series image, the region of interest, and the region-of-interest information can be acquired appropriately.

The coordinates calculating unit preferably calculates one or more sets of coordinates of interest on a contour of a polygon or circle that surrounds the region of interest. Thus, the one or more sets of coordinates of interest can be calculated appropriately.

The coordinates calculating unit preferably calculates, as the one or more sets of coordinates of interest, sets of coordinates of vertexes of the polygon. Thus, the one or more sets of coordinates of interest can be calculated appropriately.

The coordinates calculating unit preferably calculates, as the one or more sets of coordinates of interest, sets of coordinates of midpoints of sides of the polygon. Thus, the one or more sets of coordinates of interest can be calculated appropriately.

The polygon is preferably a square. Thus, the one or more sets of coordinates of interest can be calculated appropriately.

The coordinates calculating unit preferably calculates, as the one or more sets of coordinates of interest, sets of coordinates of points at which a circumference of the circle is equally divided into a plurality of parts. Thus, the one or more sets of coordinates of interest can be calculated appropriately.

The reporting information display control unit preferably superposes the figure on the time-series image by rotating or reversing a single figure. Thus, the one or more sets of coordinates of interest can be reported appropriately.

The reporting information display control unit preferably changes a color or concentration of the figure in accordance with the size of the region of interest. Thus, the one or more sets of coordinates of interest can be reported appropriately.

The medical image processing apparatus preferably further includes a feature quantity calculating unit that calculates an image feature quantity of the region of interest or a periphery of the region of interest, in which a color or concentration of the figure is preferably changed in accordance with the image feature quantity. Thus, the one or more sets of coordinates of interest can be reported appropriately.

The feature quantity calculating unit preferably calculates the image feature quantity based on at least one of color information, luminance information, or a contrast. Thus, the image feature quantity can be calculated appropriately.

If the region of interest is a plurality of regions of interest, the reporting information display control unit preferably assigns figures having different shapes from each other to the plurality of regions of interest. Thus, a plurality of sets of coordinates of interest can be reported appropriately.

If the region of interest is a plurality of regions of interest, the reporting information display control unit preferably assigns figures having different colors or concentrations from each other to the plurality of regions of interest. Thus, a plurality of sets of coordinates of interest can be reported appropriately.

If an interval between a plurality of sets of coordinates of interest is less than or equal to a threshold value corresponding to the size of the figure, the reporting information display control unit preferably joins a plurality of figures to each other, and, if the interval is greater than the threshold value, the reporting information display control unit preferably separates the plurality of figures away from each other in accordance with the interval between the sets of coordinates of interest. Thus, the sets of coordinates of interest can be reported appropriately.

The size of the region of interest preferably includes at least one of an area of the region of interest or a long side of a rectangle circumscribed about the region of interest. Thus, a figure of an appropriate size can be superposed.

The size of the figure preferably includes at least one of an area or a circumference length of the figure. Thus, a figure of an appropriate size can be superposed.

The reporting information display control unit preferably changes the size of the figure continuously in accordance with a continuous change of the size of the region of interest. Thus, a figure of an appropriate size can be superposed.

The reporting information display control unit preferably changes the size of the figure in a stepwise manner in accordance with a continuous change of the size of the region of interest. Thus, a figure of an appropriate size can be superposed.

In order to achieve the above object, an endoscope system according to an aspect is an endoscope system including: the above medical image processing apparatus; an endoscope that captures the time-series image; and a display control unit that causes a display to display the time-series image captured by the endoscope, in which the reporting information display control unit superposes and displays the figure for reporting the region of interest on the time-series image displayed on the display.

According to this aspect, the region of interest can be reported without interrupting observation of the time-series image.

In order to achieve the above object, a non-transitory computer-readable recording medium according to an aspect causes a computer to execute a medical image processing method upon the computer reading a command stored in the recording medium, the medical image processing method including: a coordinates calculating step for calculating, based on region-of-interest information indicating a region of interest in a time-series image, one or more sets of coordinates of interest indicating a position of the region of interest in the time-series image; and a reporting information display control step for superposing, based on the one or more sets of coordinates of interest, a figure on the time-series image, in which, in the reporting information display control step, a size of the figure is increased, and a ratio of the size of the figure to a size of the region of interest is decreased, as the size of the region of interest is larger.

According to this aspect, the region of interest can be reported without interrupting observation of the time-series image.

According to the present invention, the region of interest can be reported without interrupting observation of the time-series image.

### BRIEF DESCRIPTION OF THE DRAWINGS

Fig. 1 illustrates an overview of an overall configuration of an endoscope system;
Fig. 2 is a block diagram illustrating an electric configuration of a medical image processing apparatus;
Fig. 3 is a diagram for describing a coordinates calculating unit and a reporting information display control unit;
Fig. 4 is a graph illustrating a relationship between a size of a region of interest and necessity for a report;
Fig. 5 is a flowchart illustrating each process of a medical image processing method;
Fig. 6 illustrates an image and reporting information displayed on a display;
Fig. 7 illustrates an image and reporting information displayed on the display after the time has passed from the state illustrated in Fig. 6;
Fig. 8 illustrates an image and reporting information displayed on the display after the time has passed from the state illustrated in Fig. 7;
Fig. 9 illustrates an image and reporting information displayed on the display after the time has passed from the state illustrated in Fig. 8;
Fig. 10 is a graph illustrating a size of a figure with respect to the size of the region of interest and a ratio of the size of the figure to the size of the region of interest;
Fig. 11 is a graph illustrating a size of a figure with respect to the size of the region of interest and a ratio of the size of the figure to the size of the region of interest;
Figs. 12A to 12E illustrate five types of reporting information applied if a polygon having a symmetric shape that surrounds a region of interest is a square;
Fig. 13 illustrates two types of reporting information displayed on a contour of a circle that surrounds the region of interest;
Fig. 14 illustrates six types of reporting information in which positions of tips of a figure are arranged on a contour of a square having a symmetric shape that surrounds the region of interest;
Fig. 15 illustrates four types of reporting information in which each figure is arranged on a contour of a triangle having a symmetric shape that surrounds the region of interest;
Fig. 16A illustrates reporting information constituted by two figures, Fig. 16B illustrates reporting information constituted by five figures, and Fig. 16C illustrates reporting information constituted by six figures;
Fig. 17 illustrates reporting information constituted by four figures; and
Fig. 18 illustrates another embodiment of an image and reporting information displayed on the display.

### DESCRIPTION OF THE PREFERRED EMBODIMENTS

Hereinafter, preferred embodiments will be described in detail with reference to the accompanying drawings.

### Overall Configuration of Endoscope System

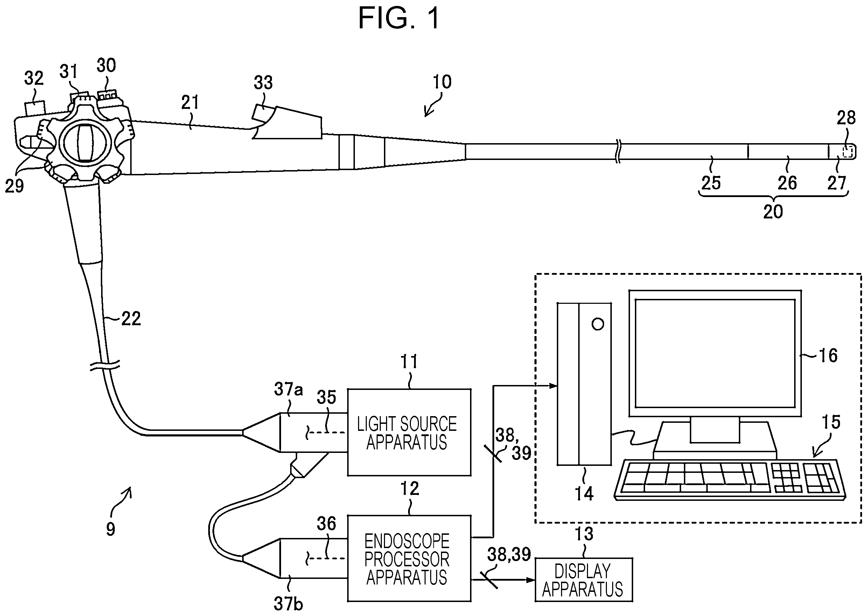

Fig. 1 illustrates an overview of an overall configuration of an endoscope system 9 including a medical image processing apparatus according to an embodiment. As illustrated in Fig. 1, the endoscope system 9 includes an endoscope 10, which is an electronic endoscope, a light source apparatus 11, an endoscope processor apparatus 12, a display apparatus 13, a medical image processing apparatus 14, an operating unit 15, and a display 16.

The endoscope 10 corresponds to a time-series image acquiring unit that acquires a time-series image including a photographic subject image and is a flexible endoscope, for example. The endoscope 10 has an insertion part 20, a handheld operating unit 21, and a universal cord 22. The insertion part 20 is inserted into a subject and has a distal end and a base end. The handheld operating unit 21 is disposed continuously with the base end side of the insertion part 20 and held by a surgeon to perform various operations. The universal cord 22 is disposed continuously with the handheld operating unit 21.

The entire insertion part 20 is formed to have a small diameter and an elongated shape. The insertion part 20 is constituted by a soft part 25, a bending part 26, and a distal end part 27, which are disposed continuously with each other in this order from the base end side to the distal end side. The soft part 25 has flexibility. The bending part 26 is bendable by an operation of the handheld operating unit 21. An imaging optical system (objective lens), an imaging element 28, and the like, which are not illustrated, are incorporated in the distal end part 27.

The imaging element 28 is an imaging element of a complementary metal oxide semiconductor (CMOS) type or a charge coupled device (CCD) type. Image light of a part to be observed is incident on an imaging surface of the imaging element 28 through an observation window and the objective lens. The observation window, which is not illustrated, is open on a distal end surface of the distal end part 27, and the objective lens, which is not illustrated, is disposed behind the observation window. The imaging element 28 captures the image light of the part to be observed, which is incident on the imaging surface (converts the image light into an electric signal) and outputs an image signal.

The handheld operating unit 21 is provided with various operating members to be operated by a surgeon. Specifically, the handheld operating unit 21 is provided with two types of bending operation knobs 29 to be used for a bending operation of the bending part 26, an air/water supply button 30 for air supply/water supply operations, and a suction button 31 for a suction operation. The handheld operating unit 21 is further provided with a still image pick-up command unit 32 for issuing a command for capturing a still image 39 of the part to be observed and a treatment tool introduction port 33 for inserting a treatment tool (not illustrated) into a treatment tool insertion path (not illustrated) that penetrates through the insertion part 20.

The universal cord 22 is a connection cord for connecting the endoscope 10 to the light source apparatus 11. The universal cord 22 contains a light guide 35 that penetrates through the insertion part 20, a signal cable 36, and a fluid tube (not illustrated). In addition, an end portion of the universal cord 22 is provided with a connector 37a that is connected to the light source apparatus 11 and a connector 37b that branches off from the connector 37a and is connected to the endoscope processor apparatus 12.

Since the connector 37a is connected to the light source apparatus 11, the light guide 35 and the fluid tube (not illustrated) are inserted into the light source apparatus 11. Thus, through the light guide 35 and the fluid tube (not illustrated), necessary illumination light, water, and gas are supplied from the light source apparatus 11 to the endoscope 10. As a result, the part to be observed is irradiated with the illumination light from an illumination window (not illustrated) on the distal end surface of the distal end part 27. In accordance with a pressing operation on the above-described air/water supply button 30, the gas or water is injected from an air/water supply nozzle (not illustrated) on the distal end surface of the distal end part 27 to the observation window (not illustrated) on the distal end surface.

Since the connector 37b is connected to the endoscope processor apparatus 12, the signal cable 36 is electrically connected to the endoscope processor apparatus 12. Thus, through the signal cable 36, an image signal of the part to be observed is output from the imaging element 28 of the endoscope 10 to the endoscope processor apparatus 12, and also, a control signal is output from the endoscope processor apparatus 12 to the endoscope 10.

The light source apparatus 11 supplies the illumination light through the connector 37a to the light guide 35 of the endoscope 10. As the illumination light, light in various wavelength ranges in accordance with an observation purpose, such as white light (light in a white wavelength range or light in a plurality of wavelength ranges), light in one or more specific wavelength ranges, or a combination thereof is selected. Note that the specific wavelength range is narrower than the white wavelength range.

A first example of the specific wavelength range is, for example, a blue range or a green range in a visible range. The wavelength range of the first example includes a wavelength range of greater than or equal to 390 nm and less than or equal to 450 nm or greater than or equal to 530 nm and less than or equal to 550 nm, and light of the first example has a peak wavelength in the wavelength range of greater than or equal to 390 nm and less than or equal to 450 nm or greater than or equal to 530 nm and less than or equal to 550 nm.

A second example of the specific wavelength range is, for example, a red range in a visible range. The wavelength range of the second example includes a wavelength range of greater than or equal to 585 nm and less than or equal to 615 nm or greater than or equal to 610 nm and less than or equal to 730 nm, and light of the second example has a peak wavelength in the wavelength range of greater than or equal to 585 nm and less than or equal to 615 nm or greater than or equal to 610 nm and less than or equal to 730 nm.

A third example of the specific wavelength range includes a wavelength range in which oxidized hemoglobin and reduced hemoglobin have different absorption coefficients, and light of the third example has a peak wavelength in the wavelength range in which oxidized hemoglobin and reduced hemoglobin have different absorption coefficients. The wavelength range of the third example includes a wavelength range of 400 ± 10 nm, 440 ± 10 nm, 470 ± 10 nm, or greater than or equal to 600 nm and less than or equal to 750 nm, and light of the third example has a peak wavelength in the wavelength range of 400 ± 10 nm, 440 ± 10 nm, 470 ± 10 nm, or greater than or equal to 600 nm and less than or equal to 750 nm.

A fourth example of the specific wavelength range is the wavelength range (from 390 nm to 470 nm) of excitation light that is used for observing fluorescence (fluorescence observation) emitted by a fluorescent material in a living body and that excites the fluorescent material.

A fifth example of the specific wavelength range is the wavelength range of infrared light. The wavelength range of the fifth example includes a wavelength range of greater than or equal to 790 nm and less than or equal to 820 nm or greater than or equal to 905 nm and less than or equal to 970 nm, and light of the fifth example has a peak wavelength in the wavelength range of greater than or equal to 790 nm and less than or equal to 820 nm or greater than or equal to 905 nm and less than or equal to 970 nm.

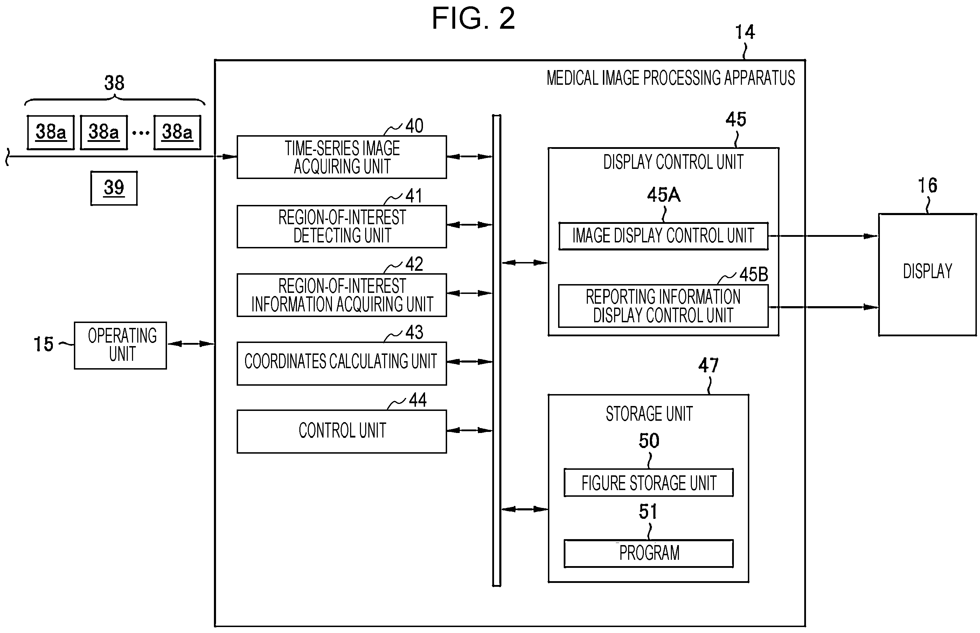

The endoscope processor apparatus 12 controls operations of the endoscope 10 through the connector 37b and the signal cable 36. In addition, based on the image signal acquired from the imaging element 28 of the endoscope 10 through the connector 37b and the signal cable 36, the endoscope processor apparatus 12 generates a moving image 38 that is a time-series image (endoscopic image) formed of time-series frame images 38a (see Fig. 2) including the photographic subject image. Furthermore, if the still image pick-up command unit 32 is operated in the handheld operating unit 21 of the endoscope 10, concurrently with the generation of the moving image 38, the endoscope processor apparatus 12 acquires one frame image 38a in the moving image 38 as the still image 39 in accordance with the timing of an imaging command.

The moving image 38 and the still image 39 are medical images obtained by capturing images of the inside of the subject, that is, a living body. In addition, if the moving image 38 and the still image 39 are images obtained with the above-described light in the specific wavelength range (special light), both are special light images. In addition, the endoscope processor apparatus 12 outputs the generated moving image 38 and the still image 39 to each of the display apparatus 13 and the medical image processing apparatus 14.

Note that the endoscope processor apparatus 12 may generate (acquire) the special light image having information on the above-described specific wavelength range, based on a usual light image obtained with the above-described white light. In this case, the endoscope processor apparatus 12 functions as a special light image acquiring unit. Then, the endoscope processor apparatus 12 obtains a signal in the specific wavelength range by performing calculation based on RGB color information of red, green, and blue or CMY color information of cyan, magenta, and yellow included in the usual light image.

Based on, for example, at least one of the usual light image obtained with the above-described white light or the special light image obtained with the above-described light in the specific wavelength range (special light), the endoscope processor apparatus 12 may generate a feature quantity image such as a known oxygen saturation image. In this case, the endoscope processor apparatus 12 functions as a feature quantity image generating unit. Note that each of the moving image 38 and the still image 39 including the above-described in-living-body image, the usual light image, the special light image, and the feature quantity image is a medical image obtained by converting results of imaging or measuring of a human body into an image for the purpose of image diagnosis or inspection.

The display apparatus 13 is connected to the endoscope processor apparatus 12 and displays the moving image 38 and the still image 39 input from the endoscope processor apparatus 12. A surgeon (physician) operates the insertion part 20 back and forth, for example, while viewing the moving image 38 displayed on the display apparatus 13, and, if a lesion or the like is found at the part to be observed, the surgeon (physician) operates the still image pick-up command unit 32 to capture a still image of the part to be observed for diagnosis, biopsy, or the like.

### Configuration of Medical Image Processing Apparatus

The medical image processing apparatus 14 mainly reports a region of interest included in a time-series image to a surgeon, and, for example, a personal computer is used as the medical image processing apparatus 14 in this embodiment. In addition, a keyboard, a mouse, or the like connected to the personal computer via wired or wireless connection is used as the operating unit 15, and any monitor, such as a liquid crystal monitor that can be connected to the personal computer, is used as the display 16.

Fig. 2 is a block diagram illustrating an electric configuration of the medical image processing apparatus 14. The medical image processing apparatus 14 illustrated in Fig. 2 is mainly constituted by a time-series image acquiring unit 40, a region-of-interest detecting unit 41, a region-of-interest information acquiring unit 42, a coordinates calculating unit 43, a control unit 44, a display control unit 45, and a storage unit 47.

Based on a program (medical image processing program) 51 stored in the storage unit 47, the control unit 44 generally controls the time-series image acquiring unit 40, the region-of-interest detecting unit 41, the region-of-interest information acquiring unit 42, the coordinates calculating unit 43, and the display control unit 45 and functions as part of these units.

The storage unit 47 is a part that stores detection results obtained by the region-of-interest detecting unit 41 and stores a captured still image 39, and also stores information or the like related to various controls of a figure storage unit 50 that stores figures constituting the reporting information, a program 51, and the medical image processing apparatus 14.

The time-series image acquiring unit 40 acquires, from the endoscope processor apparatus 12 (Fig. 1), the moving image 38 (moving image 38 captured by the endoscope 10 in this example), formed of the time-series frame images 38a including a photographic subject image, by using an image input/output interface, which is not illustrated, connected to the endoscope processor apparatus 12 via wired or wireless connection. In addition, if the above-described still image 39 is captured while the moving image 38 is being captured by the endoscope 10, the time-series image acquiring unit 40 acquires the moving image 38 and the still image 39 from the endoscope processor apparatus 12.

Note that, instead of directly acquiring the moving image 38 from the endoscope processor apparatus 12, the time-series image acquiring unit 40 may acquire the moving image 38 via any information storage medium, such as a memory card or a hard disk apparatus. In addition, the time-series image acquiring unit 40 may acquire, via the Internet, the moving image 38 uploaded on a server, database, or the like on the Internet.

The region-of-interest detecting unit 41 is a part that detects a region of interest from the moving image 38 captured during observation of a body cavity. The region-of-interest detecting unit 41 calculates a feature quantity (an example of an image feature quantity) of the frame images 38a (or the frame images 38a decimated at certain intervals) of the moving image 38, includes a convolutional neural network (CNN) that performs recognition processing of the region of interest within an image, and calculates a feature quantity from color information, a pixel value gradient, or the like within the image. By using the calculated feature quantity, the region-of-interest detecting unit 41 detects the region of interest such as a lesion in the image.

As examples of the region of interest, there are a polyp, cancer, a colon diverticulum, inflammation, an endoscopic mucosal resection (EMR) scar, an endoscopic submucosal dissection (ESD) scar, a clipped part, a bleeding point, perforation, an atypical vessel, a treatment tool, and the like.

The region-of-interest detecting unit 41 can further acquire a recognition result of, for example, category classification as to whether the detected region of interest belongs to which of a plurality of categories about the lesion, such as "tumorous", "non-tumorous", and "others".

Note that the region-of-interest detecting unit 41 is not limited to the one that detects the region of interest by the CNN, but may detect the region of interest by analyzing a feature quantity such as the color, pixel value gradient, shape, or size the image through image processing.

If the region-of-interest detecting unit 41 detects the region of interest, the region-of-interest information acquiring unit 42 acquires region-of-interest information indicating the region of interest from the region-of-interest detecting unit 41. The region-of-interest information can be, for example, information of coordinates of a contour of the region of interest in the image.

The coordinates calculating unit 43 acquires the region-of-interest information from the region-of-interest information acquiring unit 42 and, based on the acquired region-of-interest information, calculates one or more sets of coordinates of interest indicating the position of the region of interest in the moving image 38. The coordinates calculating unit 43 calculates, for example, one or more sets of coordinates of interest on the contour of a polygon or a circle that surrounds a region of interest. As the one or more sets of coordinates of interest, sets of coordinates of vertexes of the polygon or sets of coordinates of midpoints of sides of the polygon may be calculated, or sets coordinates of points at which a circumference of the circle is equally divided into a plurality of parts may be calculated.

The display control unit 45 includes an image display control unit 45A and a reporting information display control unit 45B. The image display control unit 45A outputs the moving image 38 acquired by the time-series image acquiring unit 40 to the display 16 and causes the display 16 to display the moving image 38. Based on the sets of coordinates of interest calculated by the coordinates calculating unit 43, the reporting information display control unit 45B outputs, to the display 16, reporting information formed of a plurality of figures which are figures for reporting a region of interest and the number of which is equal to that of a plurality of sets of coordinates of interest, and superposes the reporting information on the moving image 38 displayed on the display 16.

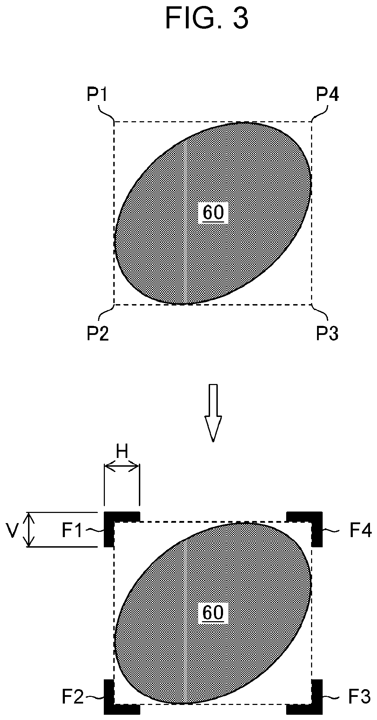

Fig. 3 is a diagram for describing the coordinates calculating unit 43 and the reporting information display control unit 45B. In this example, the coordinates calculating unit 43 calculates, as sets of coordinates of interest P1 to P4, sets of coordinates of four vertexes of a square (rectangle indicated by the broken line) in which a region of interest 60 is inscribed as illustrated in Fig. 3.

If the sets of coordinates of interest P1 to P4 are input, the reporting information display control unit 45B superposes and displays the reporting information constituted by the following figures. That is, from the set of coordinates of interest P1, the reporting information display control unit 45B generates a line segment H directed rightward in the horizontal direction and a line segment V directed downward in the vertical direction in Fig. 3 and generates an L-shaped figure F1 formed of these line segments H and V. In substantially the same manner, for the other sets of coordinates of interest P2, P3, and P4, the reporting information display control unit 45B generates figures F2, F3, and F4 corresponding to the sets of coordinates of interest P2, P3, and P4, respectively.

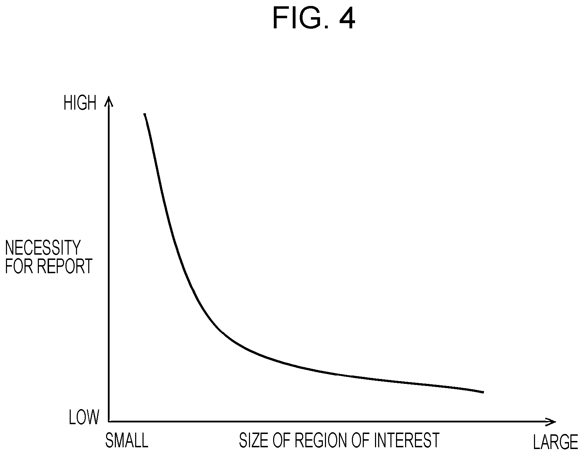

Fig. 4 is a graph illustrating a relationship between the size of the region of interest and necessity for a report. As illustrated in Fig. 4, a surgeon is more likely to find the region of interest if the size of the region of interest is larger, and thus, the necessity for a report is relatively decreased. In addition, a surgeon is less likely to find the region of interest if the size of the region of interest is smaller, and thus, the necessity for a report is relatively increased.

If the size of a figure to be superposed is excessively large relative to increase in the size of the region of interest despite relative decrease in the necessity for a report, the degree of influence of the figure to be superposed on a screen is increased, and observation is more adversely influenced. That is, excessive emphasis processing is performed despite a low degree of importance.

On the other hand, if the size of the figure to be superposed is excessively small in proportion to decrease in the size of the region of interest despite relative increase in the necessity for a report, the figure to be superposed on a screen is less noticeable, which may result in missing of the region of interest.

Thus, the size of the figure that is the reporting information needs to be set to an appropriate size in accordance with the size of the region of interest. In this embodiment, as the size of the region of interest is larger, the reporting information display control unit 45B increases the size of the figures F1 to F4 and decreases the ratio of the size of the figures F1 to F4 to the size of the region of interest. This can relatively decrease the degree of influence of the figure to be superposed in accordance with increase in the region of interest. Thus, the region of interest can be reported without interrupting observation of a time-series image.

The size of the region of interest may be the area of the region of interest itself or may be the area of a polygon or a circle in which the region of interest is inscribed. The size of the region of interest may include at least one of the area of the region of interest or a long side of a rectangle circumscribed about the region of interest.

The size of each of the figures F1 to F4 may be the length of at least one of the line segment H or the line segment V or may be the thickness of at least one of the line segment H or the line segment V. The size of each of the figures F1 to F4 may include at least one of the area or the circumference length of the figures F1 to F4.

The reporting information display control unit 45B outputs image data indicating the generated figures F1 to F4 to the display 16 and superposes and displays the reporting information constituted by the figures F1 to F4 on the image. By the figures F1 to F4 being displayed on the display 16, the region of interest in the image being displayed can be reported to a surgeon.

### Medical Image Processing Method

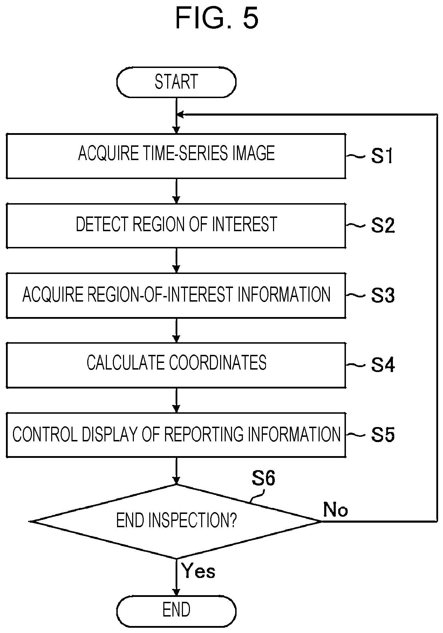

A medical image processing method using the endoscope system 9 is described. Fig. 5 is a flowchart illustrating each process of the medical image processing method. The medical image processing method includes a time-series image acquisition step (step S1), a region-of-interest detection step (step S2), a region-of-interest information acquisition step (step S3), a coordinates calculation step (step S4), and a reporting information display control step (step S5).

In step S1, the time-series image acquiring unit 40 acquires the moving image 38 captured by the endoscope 10.

In step S2, the region-of-interest detecting unit 41 detects a region of interest from the moving image 38 acquired in step S1.

In step S3, the region-of-interest information acquiring unit 42 acquires coordinates information of the contour of the region of interest in an image as region-of-interest information indicating the region of interest from the region of interest detected in step S2.

In step S4, the coordinates calculating unit 43 calculates, based on the region-of-interest information acquired in step S3, four sets of coordinates of interest at vertexes of a square that surrounds the region of interest as one or more sets of coordinates of interest indicating the position of the region of interest.

In step S5, the medical image processing apparatus 14 superposes figures on a time-series image based on the sets of coordinates of interest. Herein, the image display control unit 45A causes the display 16 to display the moving image 38. In addition, the reporting information display control unit 45B superposes the figures at the positions of the sets of coordinates of interest for the moving image 38 displayed on the display 16. As described above, the reporting information display control unit 45B increases the size of the figures and decreases the ratio of the size of the figures to the size of the region of interest, as the size of the region of interest is larger.

In step S6, the control unit 44 determines if an endoscope inspection ends. If the inspection ends, the process in this flowchart ends. If the inspection does not end, substantially the same process from step S1 is repeated.

### Display Examples of Reporting Information (Figures)

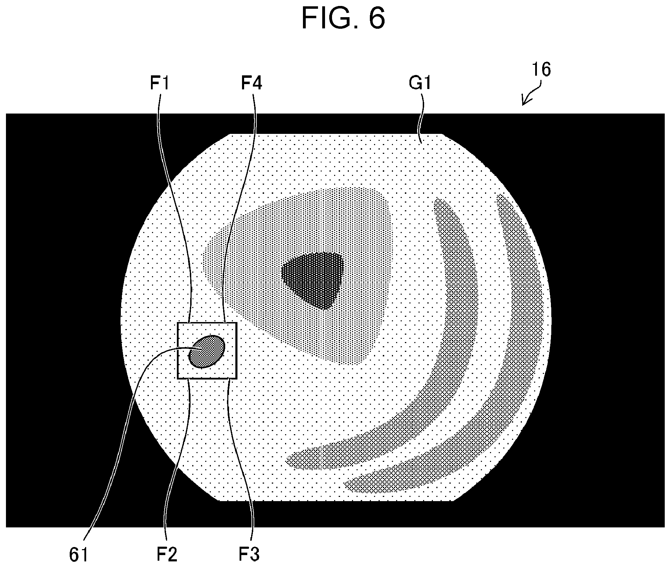

Figs. 6 to 9 each illustrate examples of the image and the reporting information (figures) displayed on the display 16, and the images and the reporting information are in a state where time elapses from Fig. 6 to Fig. 9.

That is, images G1 to G4 illustrated in Figs. 6 to 9 illustrate, in time series order, the frame images 38a that are parts of the moving image 38 obtained if the distal end of the endoscope 10 is made to gradually approach the same region of interest. The images G1 to G4 are images having the same size; a region of interest 61, a region of interest 62, a region of interest 63, and a region of interest 64 are detected from the image G1, the image G2, the image G3, and the image G4, respectively.

In the image G1 illustrated in Fig. 6, since the region of interest 61 is away from the distal end of the endoscope 10, the captured image of the region of interest 61 is small. The region of interest 61 has a size R1, and the figures F1 to F4 superposed and displayed on the image G1 have a size L1.

In the image G1, the region of interest 61 is small and the interval between the sets of coordinates of interest is less than or equal to a predetermined interval (e.g., the length of 48 pixels in a case where the width of the image is 1280 pixels and the height thereof is 1024 pixels) corresponding to the size of the figures F1 to F4 (example of a case of less than or equal to a threshold value), and thus, the figures F1 to F4 are joined to one another to constitute a rectangular frame. Note that for the reporting information constituted by the figures F1 to F4, the length of each side is preferably limited so as not to be less than or equal to the predetermined interval (the length of 48 pixels in this example) corresponding to the size of the figures F1 to F4. Otherwise, the reporting information is not noticeable due to an extremely small size, and a small region of interest may be more likely to be missed.

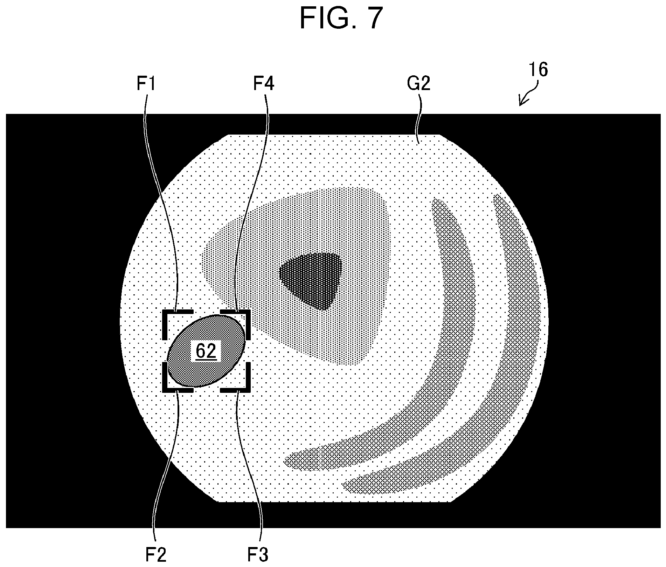

The region of interest 62 in the image G2 illustrated in Fig. 7 is larger than the region of interest 61. If the region of interest 62 has a size R2, the relationship between R1 and R2 is R1 < R2. In addition, if the figures F1 to F4 superposed and displayed on the image G2 have a size L2, the relationship between L1 and L2 is L1 < L2. Furthermore, the size of the regions of interest and the size of the figures have a relationship L1/R1 > L2/R2.

Note that, in this example, the figures F1 to F4 have different sizes by having different thicknesses of the line segment H (width in the vertical direction) and the line segment V (width in the horizontal direction) (that is, different areas and circumference lengths).

Since the region of interest 62 is larger than the region of interest 61 and the interval between the sets of coordinates of interest is also increased (example of a case of greater than the threshold value), the figures F1 to F4 superposed and displayed on the image G2 are separated from one another in accordance with the size of the region of interest 62. Although the figures F1 to F4 are separated from one another in the image G2, the region of interest 62 is comparatively small, and the figures F1 to F4 become closer to one another and more noticeable.

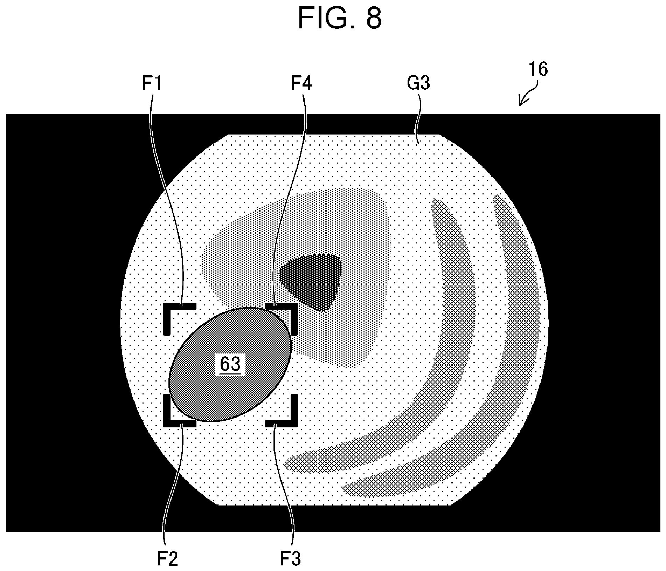

On the other hand, the region of interest 63 in the image G3 illustrated in Fig. 8 is larger than the region of interest 62. If the region of interest 63 has a size R3, the relationship between R2 and R3 is R2 < R3. In addition, if the figures F1 to F4 superposed and displayed on the image G3 have a size L3, the relationship between L2 and L3 is L2 < L3. Furthermore, the size of the regions of interest and the size of the figures have a relationship L2/R2 > L3/R3.

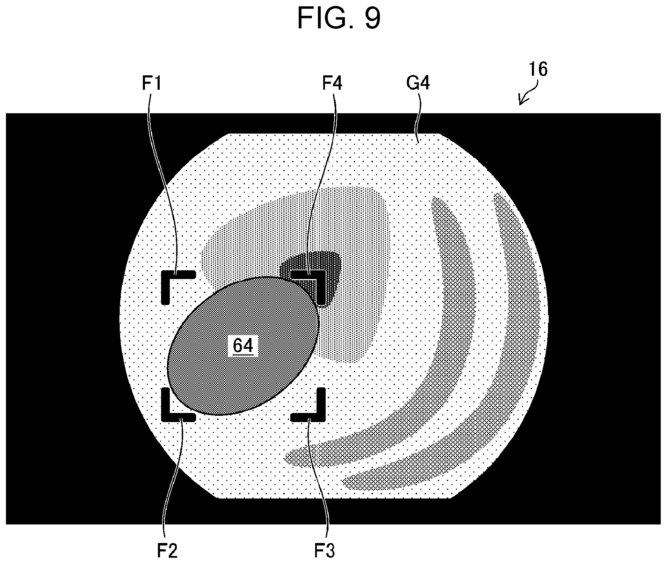

In addition, the region of interest 64 in the image G4 illustrated in Fig. 9 is larger than the region of interest 63. If the region of interest 64 has a size R4, the relationship between R3 and R4 is R3 < R4. In addition, if the figures F1 to F4 superposed and displayed on the image G4 have a size L4, the relationship between L3 and L4 is L3 < L4. Furthermore, the size of the regions of interest and the size of the figures have a relationship L3/R3 > L4/R4.

The figures F1 to F4 in the image G3 and the figures F1 to F4 in the image G4 are separated away from one another in accordance with the size of the regions of interest 63 and 64. Thus, the figures F1 to F4 become more separated away from one another in accordance with the size of the regions of interest 63 and 64, and the figures F1 to F4 become more away from one another and less noticeable, and comparison between the region of interest 63 with its peripheral region and between the region of interest 64 and its peripheral region is not interrupted.

In this manner, the size of the regions of interest 61, 62, 63, and 64 in the images have a relationship R1 < R2 < R3 < R4. In contrast, the size of the figures F1 to F4 superposed on the regions of interest 61, 62, 63, and 64 have a relationship L1 < L2 < L3 < L4. That is, as the size of the region of interest is increased, the size of the figure is increased.

Furthermore, the size of the regions of interest and the size of the figures have a relationship L1/R1 > L2/R2 > L3/R3 > L4/R4. That is, as the size of the region of interest is increased, the ratio of the size of the figure to the size of the region of interest is lower.

Note that the coordinates calculating unit 43 illustrated in Fig. 2 calculates, as the sets of coordinates of interest P1 to P4, the sets of coordinates of the four vertexes of the rectangle in which the region of interest 60 is inscribed as illustrated in Fig. 3. Without limitation to this, the rectangle in which the region of interest 60 is inscribed may be enlarged at a certain magnification (e.g., 1.2 times), and the vertexes of the enlarged rectangle may be calculated as the sets of coordinates of interest, or the sets of coordinates of the four vertexes of the rectangle in which the region of interest 60 is inscribed may be moved by a certain amount in the direction to be separated away from one another, and the moved sets of coordinates may be calculated as the sets of coordinates of interest.

In addition, the reporting information display control unit 45B is not limited to the case of generating the figures F1 to F4 in accordance with the size of the region of interest. The figures F1 to F4 in accordance with the size of the region of interest may be read out from the figure storage unit 50 that stores the figures F1 to F4 (icon images) constituting the reporting information, and based on the sets of coordinates of interest P1 to P4, the figures F1 to F4 may be arranged at positions corresponding to the sets of coordinates of interest P1 to P4 so that the reporting information constituted by the figures F1 to F4 can be superposed on an image.

Note that the reporting information display control unit 45B may change the size of the figure continuously in accordance with a continuous change of the size of the region of interest, or may change the size of the figure in a stepwise manner in accordance with a continuous change of the size of the region of interest.

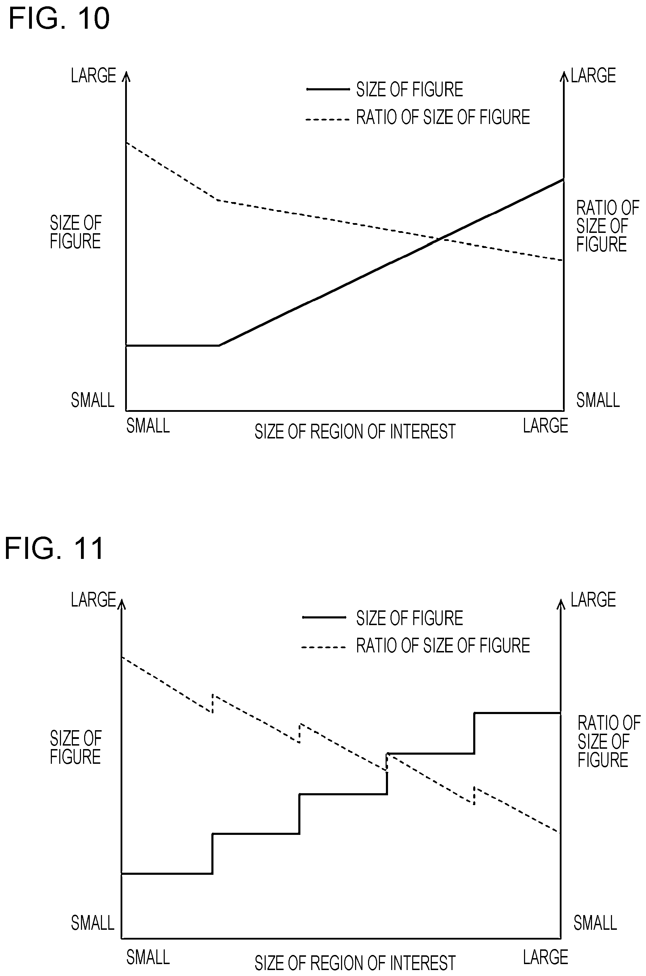

Each of Figs. 10 and 11 is a graph indicating the size of the figure with respect to the size of the region of interest and the ratio of the size of the figure to the size of the region of interest. Fig. 10 illustrates an example of a case in which the size of the figure is changed continuously in accordance with a continuous change of the size of the region of interest, and Fig. 11 illustrates an example of a case in which the size of the figure is changed in a stepwise manner in accordance with a continuous change of the size of the region of interest.

In either case, it is possible to superpose the figure of an appropriate size on the time-series image.

### Variations of Reporting Information (Figures)

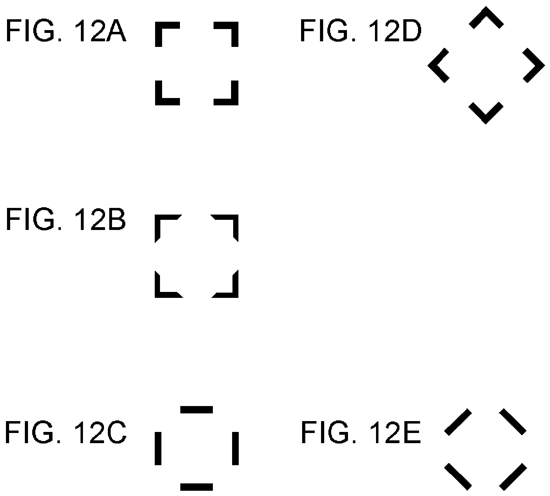

At least one figure that is reporting information may be superposed at at least one of sets of coordinates of interest, and a plurality of figures that are reporting information are preferably superposed at a plurality of sets of coordinates that surround the region of interest. Figs. 12A to 12E illustrate five types of reporting information displayed on a contour of a square having a symmetric shape that surrounds the region of interest.

The reporting information illustrated in Fig. 12A is reporting information constituted by the figures F1 to F4 illustrated in Fig. 3 and the like, and the reporting information in Fig. 12B is a modification of the reporting information in Fig. 12A.

The reporting information in Fig. 12C is applied when sets of coordinates of midpoints of sides of the rectangle that surrounds the region of interest are set as the sets of coordinates of interest and is constituted by figures of line segments of a certain length.

Each of the reporting information in Fig. 12D and the reporting information in Fig. 12E is applied when a polygon that surrounds the region of interest is a diamond, and the reporting information in Fig. 12D is applied when the sets of coordinates of vertexes of the diamond are set as the sets of coordinates of interest whereas the reporting information in Fig. 12E is applied when the sets of coordinates of midpoints of sides of the diamond are set as the sets of coordinates of interest.

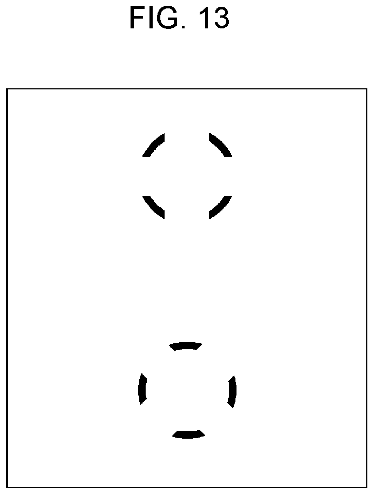

Fig. 13 illustrates two types of reporting information (figures) displayed on a contour of a circle that surrounds the region of interest. Each of the two types of reporting information illustrated in Fig. 13 is applied when sets of coordinates of points at which a circumference of a circle that surrounds the region of interest is equally divided into a plurality of parts (points at which quarters are obtained in the example in Fig. 13) are set as the sets of coordinates of interest and is constituted by four arc figures.

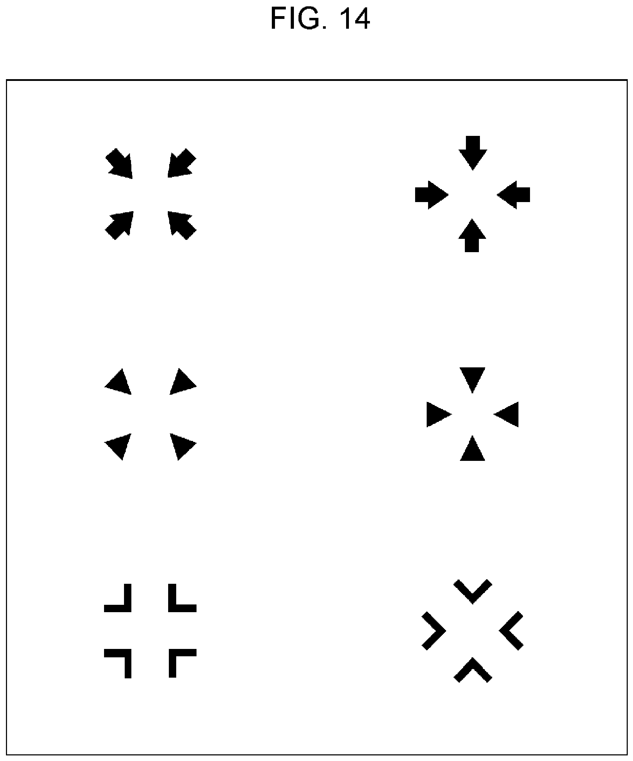

Fig. 14 illustrates six types of reporting information in which positions of tips of figures are arranged on a contour of a square having a symmetric shape that surrounds the region of interest. Each type of reporting information illustrated in Fig. 14 is constituted by arrows or figures having the same meaning as arrows. In the reporting information illustrated in Fig. 14, positions of tips of a plurality of arrows and the like correspond to positions of the sets of coordinates of interest, and the plurality of arrows and the like are arranged to be directed at the region of interest.

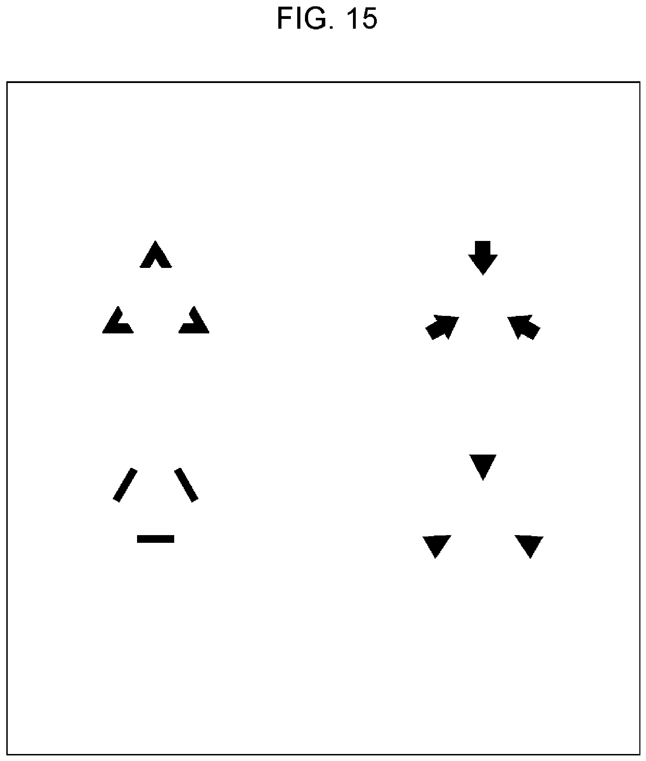

Fig. 15 illustrates four types of reporting information in which each figure is arranged on a contour of a triangle having a symmetric shape that surrounds the region of interest. Each type of reporting information illustrated in Fig. 15 is applied when a polygon that surrounds the region of interest is a triangle (regular triangle) and is constituted by three figures arranged by using vertexes of the regular triangle or midpoints of sides of the regular triangle as the sets of coordinates of interest.

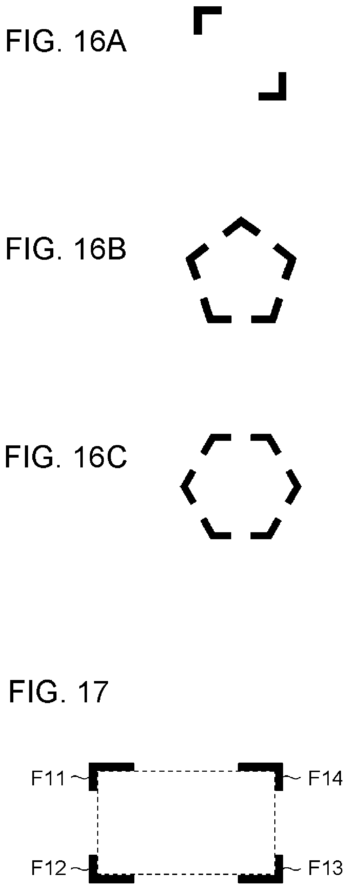

Fig. 16A illustrates reporting information constituted by two figures, Fig. 16B illustrates reporting information constituted by five figures, and Fig. 16C illustrates reporting information constituted by six figures. The reporting information illustrated in Fig. 16A is reporting information constituted by two figures arranged at diagonal positions of a rectangle that surrounds the region of interest.

The reporting information illustrated in Fig. 16B is applied when a polygon that surrounds the region of interest is a pentagon (regular pentagon), and the reporting information illustrated in Fig. 16C is applied when a polygon that surrounds the region of interest is a hexagon (regular hexagon).

The plurality of figures constituting the reporting information illustrated in Figs. 12A to 16C are figures obtained by rotating a single figure, as the plurality of figures constituting the reporting information are similar figures.

Fig. 17 illustrates reporting information constituted by four figures F11 to F14. The figures F11 and F13 illustrated in Fig. 17 are point-symmetrical figures. The figure F11 rotated 180 degrees corresponds to the figure F13. The figures F11 and F12 are line-symmetrical figures, and the figures F11 and F14 are line-symmetrical figures. The figure F11 axially reversed in the horizontal direction in Fig. 17 corresponds to the figure F12, and the figure F11 axially reversed in the vertical direction corresponds to the figure F14.

That is, the four figures F11 to F14 illustrated in Fig. 17 are figures that can be generated by rotating or reversing a single figure.

The figure storage unit 50 illustrated in Fig. 2 can store the one or more types of reporting information illustrated in Figs. 12A to 17. However, by storing only one of the plurality of figures constituting one type of reporting information, and by the reporting information display control unit 45B rotating or reversing the one of the plurality of figures constituting the reporting information, a plurality of figures constituting the reporting information can be acquired and used for a plurality of sets of coordinates of interest. That is, the plurality of figures can be generated from a single figure without individually preparing the figures.

### Color and Concentration of Reporting Information (Figures)

In addition to setting the size of the figure, in accordance with a feature quantity of the region of interest or the periphery of the region of interest calculated by the region-of-interest detecting unit 41 (example of feature quantity calculating unit), the color or concentration of the figure may be changed, and a reporting ability and the degree of influence on observation may be adjusted. For example, if the size of the region of interest is relatively small, the reporting ability is adjusted to be relatively large by implementing at least one of the following (1), (2), or (3).
(1) Change the color to a relatively high saturation color
(2) Change the color to a color with which a color difference from the color of a mucous membrane is relatively large
(3) Change the color to a relatively high concentration color
   In contrast, if the size of the region of interest is relatively large, the reporting ability is adjusted to be relatively small, and the influence on observation is adjusted to be small by implementing at least one of the following (4). (5), or (6).
(4) Change the color to a relatively low saturation color
(5) Change the color to a color with which a color difference from the color of a mucous membrane is relatively small
(6) Change the color to a relatively low concentration color

In addition, in addition to the size of the region of interest, in accordance with features of the region of interest, the color or concentration may be changed. Specifically, if the region of interest is relatively dark, by implementing at least one of the above (1), (2), or (3), the reporting ability is adjusted to be large. If the region of interest is relatively dark, a surgeon is unlikely to find the region of interest, and thus, the necessity for a report is large. Thus, by relatively increasing the visibility of the figure, it is possible to contribute to a decrease in the possibility of missing. For substantially the same reasons, if the contrast of the region of interest is relatively low, and if the saturation is relatively low, the visibility of the figure is set to be relatively increased.

If the features are reverse, by implementing at least one of the above (4). (5), or (6), the visibility is set to be relatively decreased, and the influence on observation is decreased.

The features to be referred to when changing the color or concentration of the figure may be any one of the size of the region of interest, luminance information, color information (brightness, saturation), and a contrast, or may be a combination thereof.

### Case in which Plurality of Regions of Interest Are Present

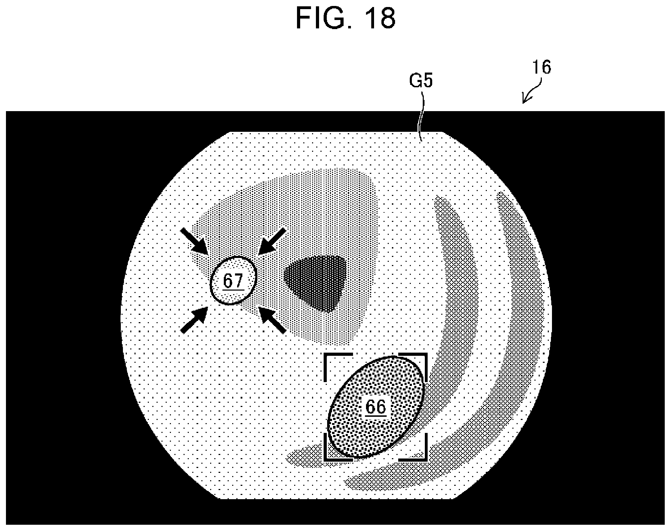

Fig. 18 illustrates another embodiment of an image and reporting information (figures) displayed on the display 16, and a plurality of regions of interest 66 and 67 are present in an image G5 illustrated in Fig. 18.

If a plurality of regions of interest are present within one frame image, the region-of-interest detecting unit 41 illustrated in Fig. 2 can detect the plurality of regions of interest 66 and 67 by the CNN.

If the region-of-interest detecting unit 41 detects the plurality of regions of interest 66 and 67, the region-of-interest information acquiring unit 42 acquires a plurality of pieces of region-of-interest information indicating the regions of interest 66 and 67 from the region-of-interest detecting unit 41. Upon acquiring the plurality of pieces of region-of-interest information from the region-of-interest information acquiring unit 42, based on the acquired plurality of pieces of region-of-interest information, the coordinates calculating unit 43 calculates a plurality of sets of coordinates of interest surrounding the regions of interest for each of the plurality of regions of interest 66 and 67.

Upon the region-of-interest information acquiring unit 42 acquiring the plurality of pieces of region-of- interest information corresponding to the plurality of regions of interest 66 and 67, the reporting information display control unit 45B adds reporting information to each of the plurality of regions of interest 66 and 67. In this case, the reporting information display control unit 45B preferably assigns the reporting information constituted by figures having different shapes to the plurality of regions of interest 66 and 67.

In the example illustrated in Fig. 18, a plurality of substantially L-shaped figures are assigned to the front-side large region of interest 66, and a plurality of arrow figures are assigned to the deep-side small region of interest 67.

Alternatively, the reporting information display control unit 45B may assign figures having different colors to the plurality of regions of interest when adding the figures to the plurality of regions of interest. In this case, the figures may have an identical shape or different shapes.

Thus, it becomes easy to distinguish the plurality of regions of interest by the figures having different shapes or different colors, and it becomes easy to visually follow the plurality of regions of interest when the plurality of regions of interest move.

### Miscellaneous

The above medical image processing method is configured as a program causing a computer to execute each step, and a non-transitory recording medium such as a compact disk-read only memory (CD-ROM) storing this program may also be configured.

Although the endoscope processor apparatus 12 and the medical image processing apparatus 14 are different apparatuses in the above embodiments, the endoscope processor apparatus 12 and the medical image processing apparatus 14 may also be constituted as an integrated apparatus, and the functions of the medical image processing apparatus 14 may be provided in the endoscope processor apparatus 12.

In addition, a hardware structure of a processing unit that performs various processes of the endoscope processor apparatus 12 and the medical image processing apparatus 14 is any of the following various processors. Various processors include a central processing unit (CPU) that is a general-purpose processor functioning as various processing units by executing software (programs), a graphics processing unit (GPU) that is a processor specialized in image processing, a programmable logic device (PLD) that is a processor in which the circuit configuration is changeable after manufacture, such as field programmable gate array (FPGA), a dedicated electric circuit that is a processor having a circuit configuration that is specially designed to execute specific processing, such as an application specific integrated circuit (ASIC), and the like.

One processing unit may be constituted by one of these various processors, or may be constituted by two or more processors of the same type or different types (e.g., a combination of a plurality of FPGAs, a combination of a CPU and an FPGA, or a combination of a CPU and a GPU). In addition, a plurality of processing units may be configured as one processor. As a first example for configuring a plurality of processing units as one processor, one or more CPUs and software may be combined to configure one processor, and this processor may function as a plurality of processing units, as typified by a computer such as a client or a server. As a second example, a processor may be used that implements the functions of the entire system including a plurality of processing units as one integrated circuit (IC) chip, as typified by a system on chip (SoC) or the like. In this manner, various processing units are constituted by one or more of various processors as a hardware structure.

Furthermore, the hardware structure of these various processors is more specifically is electric circuitry obtained by combining circuit elements such as semiconductor elements.

The technical scope of the present invention is not limited to the scope described in the above embodiments, and configurations or the like in each embodiment may be combined as appropriate between the embodiments without departing from the spirit of the present invention.

### Reference Signs List

- 9: endoscope system
- 10: endoscope
- 11: light source apparatus
- 12: endoscope processor apparatus
- 13: display apparatus
- 14: medical image processing apparatus
- 15: operating unit
- 16: display
- 20: insertion part
- 21: handheld operating unit
- 22: universal cord
- 25: soft part
- 26: bending part
- 27: distal end part
- 28: imaging element
- 29: bending operation knob
- 30: air/water supply button
- 31: suction button
- 32: still image pick-up command unit
- 33: treatment tool introduction port
- 35: light guide
- 36: signal cable
- 37a: connector
- 37b: connector
- 38: moving image
- 38a: frame image
- 39: still image
- 40: time-series image acquiring unit
- 41: region-of-interest detecting unit
- 42: region-of-interest information acquiring unit
- 43: coordinates calculating unit
- 44: control unit
- 45: display control unit
- 45A: image display control unit
- 45B: reporting information display control unit
- 47: storage unit
- 50: figure storage unit
- 51: program
- 60: region of interest
- 61: region of interest
- 62: region of interest
- 63: region of interest
- 64: region of interest
- 66: region of interest
- 67: region of interest
- F1 to F4, F11 to F14: figure
- G1 to G5: image
- H, V: line segment
- P1 to P4: set of coordinates of interest
- S1 to S6: step of medical image processing method

## Claims

1. A medical image processing apparatus (14) comprising:
a coordinates calculating unit (43) that, based on region-of-interest information indicating a region of interest (60) in a time-series image (38), calculates one or more sets of coordinates of interest (P1, P2, P3, P4) indicating a position of the region of interest (60) in the time-series image (38); and
a reporting information display control unit (45B) that, based on the one or more sets of coordinates of interest (P1, P2, P3, P4), superposes a figure (F1, F2, F3, F4) on the time-series image (38),
**characterized in that**
the reporting information display control unit (45B) increases a size of the figure (F1, F2, F3, F4) and decreases a ratio of the size of the figure (F1, F2, F3, F4) to a size of the region of interest (60) as the size of the region of interest (60) is larger.

2. The medical image processing apparatus (14) according to claim 1, further comprising:
a time-series image acquiring unit (40) that acquires the time-series image (38) including a photographic subject image;
a region-of-interest detecting unit (41) that detects the region of interest (60) from the time-series image (38) acquired by the time-series image acquiring unit (40); and
a region-of-interest information acquiring unit (42) that acquires region-of-interest information indicating the region of interest (60) from the region-of-interest detecting unit (41).

3. The medical image processing apparatus (14) according to claim 1 or 2,
wherein the coordinates calculating unit (43) calculates one or more sets of coordinates of interest (P1, P2, P3, P4) on a contour of a polygon or circle that surrounds the region of interest (60), and
preferably, the coordinates calculating unit (43) calculates, as the one or more sets of coordinates of interest (P1, P2, P3, P4), sets of coordinates of vertexes of the polygon.

4. The medical image processing apparatus (14) according to claim 3,
wherein the coordinates calculating unit (43) calculates, as the one or more sets of coordinates of interest (P1, P2, P3, P4), sets of coordinates of midpoints of sides of the polygon, and for example, the polygon is a square.

5. The medical image processing apparatus (14) according to claim 3,
wherein the coordinates calculating unit (43) calculates, as the one or more sets of coordinates of interest (P1, P2, P3, P4), sets of coordinates of points at which a circumference of the circle is equally divided into a plurality of parts.

6. The medical image processing apparatus (14) according to any one of claims 1 to 5,
wherein the reporting information display control unit (45B) superposes the figure (F1, F2, F3, F4) on the time-series image (38) by rotating or reversing a single figure (F1, F2, F3, F4).

7. The medical image processing apparatus (14) according to any one of claims 1 to 7,
wherein the reporting information display control unit (45B) changes a color or concentration of the figure (F1, F2, F3, F4) in accordance with the size of the region of interest (60).

8. The medical image processing apparatus (14) according to any one of claims 1 to 7, further comprising:
a feature quantity calculating unit that calculates an image feature quantity of the region of interest (60) or a periphery of the region of interest (60),
wherein a color or concentration of the figure (F1, F2, F3, F4) is changed in accordance with the image feature quantity, and for example, the feature quantity calculating unit calculates the image feature quantity based on at least one of color information, luminance information, or a contrast.

9. The medical image processing apparatus (14) according to any one of claims 1 to 8,
wherein, if the region of interest (60) is a plurality of regions of interest, the reporting information display control unit (45B) assigns figures (F1, F2, F3, F4) having different shapes from each other to the plurality of regions of interest, and for example, if the region of interest (60) is a plurality of regions of interest, the reporting information display control unit (45B) assigns figures (F1, F2, F3, F4) having different colors or concentrations from each other to the plurality of regions of interest, and
preferably, if an interval between a plurality of sets of coordinates of interest (P1, P2, P3, P4) is less than or equal to a threshold value corresponding to the size of the figure (F1, F2, F3, F4), the reporting information display control unit (45B) joins a plurality of figures (F1, F2, F3, F4) to each other, and, if the interval is greater than the threshold value, the reporting information display control unit (45B) separates the plurality of figures (F1, F2, F3, F4) away from each other in accordance with the interval between the sets of coordinates of interest (P1. P2, P3, P4).

10. The medical image processing apparatus (14) according to any one of claims 1 to 9,
wherein the size of the region of interest (60) includes at least one of an area of the region of interest (60) or a long side of a rectangle circumscribed about the region of interest (60).

11. The medical image processing apparatus (14) according to any one of claims 1 to 10,
wherein the size of the figure (F1, F2, F3, F4) includes at least one of an area or a circumference length of the figure (F1, F2, F3, F4).

12. The medical image processing apparatus (14) according to any one of claims 1 to 11,
wherein the reporting information display control unit (45B) changes the size of the figure (F1, F2, F3, F4) continuously in accordance with a continuous change of the size of the region of interest (60), or the reporting information display control unit (45B) changes the size of the figure (F1, F2, F3, F4) in a stepwise manner in accordance with a continuous change of the size of the region of interest (60).

13. An endoscope system (9) comprising:
the medical image processing apparatus (14) according to any one of claims 1 to 11;
an endoscope (10) that captures the time-series image (38); and
a display control unit (45) that causes a display (16) to display the time-series image (38) captured by the endoscope (10),
wherein the reporting information display control unit (45B) superposes and displays the figure (F1, F2, F3, F4) for reporting the region of interest (60) on the time-series image (38) displayed on the display (16).

14. A non-transitory computer-readable recording medium causing a computer to execute a medical image processing method upon the computer reading a command stored in the recording medium; the medical image processing method comprising:
a coordinates calculating step (S4) for calculating, based on region-of-interest information indicating a region of interest (60) in a time-series image (38), one or more sets of coordinates of interest (P1, P2, P3, P4) indicating a position of the region of interest (60) in the time-series image (38); and
a reporting information display control step (S5) for superposing, based on the one or more sets of coordinates of interest (P1, P2, P3, P4), a figure (F1, F2, F3, F4) on the time-series image (38),
**characterized in that**
in the reporting information display control step (S5), a size of the figure (F1, F2, F3, F4) is increased, and a ratio of the size of the figure (F1, F2, F3, F4) to a size of the region of interest (60) is decreased, as the size of the region of interest (60) is larger.

## Patentansprüche

1. Verarbeitungsvorrichtung (14) für medizinische Bilder, umfassend:
eine Koordinaten-Berechnungseinheit (43), die auf der Grundlage von Informationen über Bereich von Interesse, die einen Bereich von Interesse (60) in einem Zeitreihenbild (38) angeben, einen oder mehrere Sätze von Koordinaten von Interesse (P1, P2, P3, P4), die eine Position des Bereichs von Interesse (60) in dem Zeitreihenbild (38) angeben, berechnet; und
eine Berichtsinformations-Anzeigesteuereinheit (45B), die auf der Grundlage des einen oder der mehreren Sätze von Koordinaten von Interesse (P1, P2, P3, P4) eine Figur (F1, F2, F3, F4) dem Zeitreihenbild (38) überlagert,
**dadurch gekennzeichnet, dass**
die Berichtsinformations-Anzeigesteuereinheit (45B) eine Größe der Figur (F1, F2, F3, F4) vergrößert und ein Verhältnis der Größe der Figur (F1, F2, F3, F4) zu einer Größe des Bereichs von Interesse (60) verringert, wenn die Größe des Bereichs von Interesse (60) größer ist.

2. Verarbeitungsvorrichtung (14) für medizinische Bilder nach Anspruch 1, ferner umfassend:
eine Zeitreihenbild-Erfassungseinheit (40), die das Zeitreihenbild (38), das ein fotografisches Motivbild enthält, erfasst;
eine Detektionseinheit (41) für Bereich von Interesse, die den Bereich von Interesse (60) aus dem von der Zeitreihenbild-Erfassungseinheit (40) erfassten Zeitreihenbild (38) detektiert; und
eine Informationserfassungseinheit (42) für Bereich von Interesse, die Informationen über Bereich von Interesse, die den Bereich von Interesse (60) angeben, von der Detektionseinheit (41) für Bereich von Interesse erfasst.

3. Verarbeitungsvorrichtung (14) für medizinische Bilder nach Anspruch 1 oder 2,
wobei die Koordinaten-Berechnungseinheit (43) einen oder mehrere Sätze von Koordinaten von Interesse (P1, P2, P3, P4) an einer Kontur eines Polygons oder Kreises, der den Bereich von Interesse (60) umgibt, berechnet, und
die Koordinaten-Berechnungseinheit (43) bevorzugt als den einen oder die mehreren Sätze von Koordinaten von Interesse (P1, P2, P3, P4) Sätze von Koordinaten von Eckpunkten des Polygons berechnet.

4. Verarbeitungsvorrichtung (14) für medizinische Bilder nach Anspruch 3,
wobei die Koordinaten-Berechnungseinheit (43) als den einen oder die mehreren Sätze von Koordinaten von Interesse (P1, P2, P3, P4) Sätze von Koordinaten von Mittelpunkten von Seiten des Polygons berechnet und beispielsweise das Polygon ein Quadrat ist.

5. Verarbeitungsvorrichtung (14) für medizinische Bilder nach Anspruch 3,
wobei die Koordinaten-Berechnungseinheit (43) als den einen oder die mehreren Sätze von Koordinaten von Interesse (P1, P2, P3, P4) Sätze von Koordinaten von Punkten, an denen ein Umfang des Kreises in mehrere Teile gleichmäßig unterteilt ist, berechnet.

6. Verarbeitungsvorrichtung (14) für medizinische Bilder nach einem der Ansprüche 1 bis 5,
wobei die Berichtsinformations-Anzeigesteuereinheit (45B) die Figur (F1, F2, F3, F4) dem Zeitreihenbild (38) durch Drehen oder Umkehren einer einzigen Figur (F1, F2, F3, F4) überlagert.

7. Verarbeitungsvorrichtung (14) für medizinische Bilder nach einem der Ansprüche 1 bis 7,
wobei die Berichtsinformations-Anzeigesteuereinheit (45B) eine Farbe oder Konzentration der Figur (F1, F2, F3, F4) in Übereinstimmung mit der Größe des Bereichs von Interesse (60) ändert.

8. Verarbeitungsvorrichtung für medizinische Bilder (14) nach einem der Ansprüche 1 bis 7, ferner umfassend:
eine Merkmalsmengen-Berechnungseinheit, die eine Bildmerkmalsmenge des Bereichs von Interesse (60) oder eines Umfangs des Bereichs von Interesse (60) berechnet,
wobei eine Farbe oder Konzentration der Figur (F1, F2, F3, F4) in Übereinstimmung mit der Bildmerkmalsmenge geändert wird und beispielsweise die Merkmalsmengen-Berechnungseinheit die Bildmerkmalsmenge auf der Grundlage von mindestens einer von Farbinformationen, Luminanzinformationen und einem Kontrast berechnet.

9. Verarbeitungsvorrichtung (14) für medizinische Bilder nach einem der Ansprüche 1 bis 8,
wobei, wenn der Bereich von Interesse (60) mehrere Bereiche von Interesse ist, die Berichtsinformations-Anzeigesteuereinheit (45B) Figuren (F1, F2, F3, F4), die voneinander unterschiedliche Formen aufweisen, den mehreren Bereichen von Interesse zuweist und beispielsweise, wenn der Bereich von Interesse (60) mehrere Bereiche von Interesse ist, die Berichtsinformations-Anzeigesteuereinheit (45B) Figuren (F1, F2, F3, F4), die voneinander unterschiedliche Farben oder Konzentrationen aufweisen, den mehreren Bereichen von Interesse zuweist, und
wenn ein Abstand zwischen mehreren Sätzen von Koordinaten von Interesse (P1, P2, P3, P4) kleiner oder gleich einem Schwellenwert ist, der der Größe der Figur (F1, F2, F3, F4) entspricht, die Berichtsinformations-Anzeigesteuereinheit (45B) mehrere Figuren (F1, F2, F3, F4) miteinander verbindet, und wenn der Abstand größer als der Schwellenwert ist, die Berichtsinformations-Anzeigesteuereinheit (45B) die mehreren Figuren (F1, F2, F3, F4) in Übereinstimmung mit dem Abstand zwischen den Sätzen von Koordinaten von Interesse (P1, P2, P3, P4) voneinander trennt.

10. Verarbeitungsvorrichtung (14) für medizinische Bilder nach einem der Ansprüche 1 bis 9,
wobei die Größe des Bereichs von Interesse (60) mindestens eine von einer Fläche des Bereichs von Interesse (60) und einer langen Seite eines Rechtecks, das den Bereich von Interesse (60) umschreibt, enthält.

11. Verarbeitungsvorrichtung (14) für medizinische Bilder nach einem der Ansprüche 1 bis 10,
wobei die Größe der Figur (F1, F2, F3, F4) mindestens eine von einer Fläche und einer Umfangslänge der Figur (F1, F2, F3, F4) enthält.

12. Verarbeitungsvorrichtung (14) für medizinische Bilder nach einem der Ansprüche 1 bis 11,
wobei die Berichtsinformations-Anzeigesteuereinheit (45B) die Größe der Figur (F1, F2, F3, F4) kontinuierlich in Übereinstimmung mit einer kontinuierlichen Änderung der Größe des Bereichs von Interesse (60) ändert oder die Berichtsinformations-Anzeigesteuereinheit (45B) die Größe der Figur (F1, F2, F3, F4) in einer schrittweisen Weise in Übereinstimmung mit einer kontinuierlichen Änderung der Größe des Bereichs von Interesse (60) ändert.

13. Endoskopsystem (9), umfassend:
die Verarbeitungsvorrichtung (14) für medizinische Bilder nach einem der Ansprüche 1 bis 11;
ein Endoskop (10), das das Zeitreihenbild (38) aufnimmt; und
eine Anzeigesteuereinheit (45), die eine Anzeige (16) veranlasst, das von dem Endoskop (10) aufgenommene Zeitreihenbild (38) anzuzeigen,
wobei die Berichtsinformations-Anzeigesteuereinheit (45B) die Figur (F1, F2, F3, F4) zum Berichten des Bereichs von Interesse (60) dem Zeitreihenbild (38), das auf der Anzeige (16) angezeigt wird, überlagert und anzeigt.

14. Nicht flüchtiges computerlesbares Aufzeichnungsmedium, das einen Computer veranlasst, ein Verarbeitungsverfahren für medizinische Bilder auszuführen, wenn der Computer einen in dem Aufzeichnungsmedium gespeicherten Befehl liest; wobei das Verarbeitungsverfahren für medizinische Bilder umfasst:
einen Koordinaten-Berechnungsschritt (S4) zum Berechnen, auf der Grundlage von Informationen über Bereich von Interesse, die einen Bereich von Interesse (60) in einem Zeitreihenbild (38) angeben, eines oder mehrerer Sätze von Koordinaten von Interesse (P1, P2, P3, P4), die eine Position des Bereichs von Interesse (60) in dem Zeitreihenbild (38) angeben; und
einen Berichtsinformations-Anzeigesteuerschritt (S5) zum Überlagern, auf der Grundlage des einen oder der mehreren Sätze von Koordinaten von Interesse (P1, P2, P3, P4), einer Figur (F1, F2, F3, F4) dem Zeitreihenbild (38),
**dadurch gekennzeichnet, dass**
bei dem Berichtsinformations-Anzeigesteuerschritt (S5) eine Größe der Figur (F1, F2, F3, F4) vergrößert wird, und ein Verhältnis der Größe der Figur (F1, F2, F3, F4) zu einer Größe des Bereichs von Interesse (60) wird verringert, wenn die Größe des Bereichs von Interesse (60) größer ist.

## Revendications

1. Appareil de traitement d'images médicales (14) comprenant :
une unité de calcul de coordonnées (43) qui, sur la base d'informations de région d'intérêt indiquant une région d'intérêt (60) dans une image de série temporelle (38), calcule un ou plusieurs ensembles de coordonnées d'intérêt (P1, P2, P3, P4) indiquant une position de la région d'intérêt (60) dans l'image de série temporelle (38) ; et
une unité de contrôle d'affichage d'informations de rapport (45B) qui, sur la base de l'un ou plusieurs ensembles de coordonnées d'intérêt (P1, P2, P3, P4), superpose une figure (F1, F2, F3, F4) sur l'image de série temporelle (38),
**caractérisé en ce que**
l'unité de contrôle d'affichage d'informations de rapport (45B) augmente une taille de la figure (F1, F2, F3, F4) et diminue un rapport de la taille de la figure (F1, F2, F3, F4) à une taille de la région d'intérêt (60) à mesure que la taille de la région d'intérêt (60) est plus grande.

2. Appareil de traitement d'images médicales (14) selon la revendication 1, comprenant en outre :
une unité d'acquisition d'images de série temporelle (40) qui acquiert l'image de série temporelle (38) incluant une image de sujet photographique ;
une unité de détection de région d'intérêt (41) qui détecte la région d'intérêt (60) à partir de l'image de série temporelle (38) acquise par l'unité d'acquisition d'images de série temporelle (40) ; et
une unité d'acquisition d'informations de région d'intérêt (42) qui acquiert des informations de région d'intérêt indiquant la région d'intérêt (60) à partir de l'unité de détection de région d'intérêt (41).

3. Appareil de traitement d'images médicales (14) selon la revendication 1 ou la revendication 2,
dans lequel l'unité de calcul de coordonnées (43) calcule un ou plusieurs ensembles de coordonnées d'intérêt (P1, P2, P3, P4) sur un contour d'un polygone ou d'un cercle qui entoure la région d'intérêt (60), et
de préférence, l'unité de calcul de coordonnées (43) calcule, comme l'un ou plusieurs ensembles de coordonnées d'intérêt (P1, P2, P3, P4), des ensembles de coordonnées de sommets du polygone.

4. Appareil de traitement d'images médicales (14) selon la revendication 3,
dans lequel l'unité de calcul de coordonnées (43) calcule, comme l'un ou plusieurs ensembles de coordonnées d'intérêt (P1, P2, P3, P4), des ensembles de coordonnées de milieux de côtés du polygone, et par exemple, le polygone est un carré.

5. Appareil de traitement d'images médicales (14) selon la revendication 3,
dans lequel l'unité de calcul de coordonnées (43) calcule, comme l'un ou plusieurs ensembles de coordonnées d'intérêt (P1, P2, P3, P4), des ensembles de coordonnées de points auxquels une circonférence du cercle est divisée de manière égale en une pluralité de parties.

6. Appareil de traitement d'images médicales (14) selon l'une quelconque des revendications 1 à 5,
dans lequel l'unité de contrôle d'affichage d'informations de rapport (45B) superpose la figure (F1, F2, F3, F4) sur l'image de série temporelle (38) en faisant pivoter ou en inversant une seule figure (F1, F2, F3, F4).

7. Appareil de traitement d'images médicales (14) selon l'une quelconque des revendications 1 à 7,
dans lequel l'unité de contrôle d'affichage d'informations de rapport (45B) change une couleur ou une concentration de la figure (F1, F2, F3, F4) en fonction de la taille de la région d'intérêt (60).

8. Appareil de traitement d'images médicales (14) selon l'une quelconque des revendications 1 à 7, comprenant en outre :
une unité de calcul de quantité de caractéristique qui calcule une quantité de caractéristique d'image de la région d'intérêt (60) ou d'une périphérie de la région d'intérêt (60),
dans lequel une couleur ou une concentration de la figure (F1, F2, F3, F4) est changée en fonction de la quantité de caractéristique d'image, et par exemple, l'unité de calcul de quantité de caractéristique calcule la quantité de caractéristique d'image sur la base d'au moins l'une d'informations de couleur, d'informations de luminance ou d'un contraste.

9. Appareil de traitement d'images médicales (14) selon l'une quelconque des revendications 1 à 8,
dans lequel, si la région d'intérêt (60) est une pluralité de régions d'intérêt, l'unité de contrôle d'affichage d'informations de rapport (45B) attribue des figures (F1, F2, F3, F4) ayant des formes différentes les unes des autres à la pluralité de régions d'intérêt, et par exemple, si la région d'intérêt (60) est une pluralité de régions d'intérêt, l'unité de contrôle d'affichage d'informations de rapport (45B) attribue des figures (F1, F2, F3, F4) ayant des couleurs ou des concentrations différentes les unes des autres à la pluralité de régions d'intérêt, et
de préférence, si un intervalle entre une pluralité d'ensembles de coordonnées d'intérêt (P1, P2, P3, P4) est inférieur ou égal à une valeur seuil correspondant à la taille de la figure (F1, F2, F3, F4), l'unité de contrôle d'affichage d'informations de rapport (45B) joint une pluralité de figures (F1, F2, F3, F4) les unes aux autres, et, si l'intervalle est supérieur à la valeur seuil, l'unité de contrôle d'affichage d'informations de rapport (45B) sépare la pluralité de figures (F1, F2, F3, F4) les unes des autres en fonction de l'intervalle entre les ensembles de coordonnées d'intérêt (P1, P2, P3, P4).

10. Appareil de traitement d'images médicales (14) selon l'une quelconque des revendications 1 à 9,
dans lequel la taille de la région d'intérêt (60) inclut au moins l'une parmi une aire de la région d'intérêt (60) ou un côté long d'un rectangle circonscrit autour de la région d'intérêt (60).

11. Appareil de traitement d'images médicales (14) selon l'une quelconque des revendications 1 à 10,
dans lequel la taille de la figure (F1, F2, F3, F4) inclut au moins l'une parmi une aire ou une longueur de circonférence de la figure (F1, F2, F3, F4).

12. Appareil de traitement d'images médicales (14) selon l'une quelconque des revendications 1 à 11,
dans lequel l'unité de contrôle d'affichage d'informations de rapport (45B) change la taille de la figure (F1, F2, F3, F4) de manière continue en fonction d'un changement continu de la taille de la région d'intérêt (60), ou l'unité de contrôle d'affichage d'informations de rapport (45B) change la taille de la figure (F1, F2, F3, F4) de manière progressive en fonction d'un changement continu de la taille de la région d'intérêt (60).

13. Système d'endoscope (9) comprenant :
l'appareil de traitement d'images médicales (14) selon l'une quelconque des revendications 1 à 11 ;
un endoscope (10) qui capture l'image de série temporelle (38) ; et
une unité de contrôle d'affichage (45) qui amène un affichage (16) à afficher l'image de série temporelle (38) capturée par l'endoscope (10),
dans lequel l'unité de contrôle d'affichage d'informations de rapport (45B) superpose et affiche la figure (F1, F2, F3, F4) pour signaler la région d'intérêt (60) sur l'image de série temporelle (38) affichée sur l'affichage (16).

14. Support d'enregistrement non transitoire lisible par ordinateur amenant un ordinateur à exécuter un procédé de traitement d'images médicales lorsque l'ordinateur lit une commande stockée dans le support d'enregistrement ; le procédé de traitement d'images médicales comprenant :
une étape de calcul de coordonnées (S4) pour calculer, sur la base d'informations de région d'intérêt indiquant une région d'intérêt (60) dans une image de série temporelle (38), un ou plusieurs ensembles de coordonnées d'intérêt (P1, P2, P3, P4) indiquant une position de la région d'intérêt (60) dans l'image de série temporelle (38) ; et
une étape de contrôle d'affichage d'informations de rapport (S5) pour superposer, sur la base de l'un ou plusieurs ensembles de coordonnées d'intérêt (P1, P2, P3, P4), une figure (F1, F2, F3, F4) sur l'image de série temporelle (38),
**caractérisé en ce que**
dans l'étape de contrôle d'affichage d'informations de rapport (S5), une taille de la figure (F1, F2, F3, F4) est augmentée, et un rapport de la taille de la figure (F1, F2, F3, F4) à une taille de la région d'intérêt (60) est diminué, à mesure que la taille de la région d'intérêt (60) est plus grande.
